# EUROPEAN PATENT SPECIFICATION

(11) **EP 1 839 577 B1**
(45) Date of publication and mention of the grant of the patent: **12.08.2009**
(21) Application number: 07013176.8
(22) Date of filing: 25.06.2003
(51) Int. Cl.: A61B 5/15, A61B 10/00

(54) **Sensor with integrated lancet**
Sensor mit integrierter Lanzette
Capteur à lancette intégrée

(30) Priority: 25.06.2002 US 391108 P
(43) Date of publication of application: 03.10.2007
(62) Divisional of application: 03014233.5
(73) Proprietor: Bayer HealthCare, LLC, Tarrytown, NY 10591 (US)
(72) Inventor: Vreeke, Mark S., Houston Texas 77070 (US); Charlton, Steven C., Osceola Indiana 46561 (US); McClearly, Alan R., South Bend Indiana 46637 (US); Flora, Bruce A., Elkhart, Indiana 46514 (US)
(74) Representative: Burkert, Frank

(56) References cited:
- EP-A- 1 174 083
- GB-A- 2 331 935
- US-A- 4 627 445

## Description

### FIELD OF THE INVENTION

The present invention relates generally to blood monitoring devices and, more particularly, to a sensor having an integrated lance.

### BACKGROUND OF THE INVENTION

It is often necessary to quickly obtain a sample of blood and perform an analysis of the blood sample. One example of a need for quickly obtaining a sample of blood is in connection with a blood glucose monitoring system where a user must frequently use the system to monitor the user's blood glucose level.

Those who have irregular blood glucose concentration levels are often medically required to self-monitor their blood glucose concentration level. An irregular blood glucose level can be brought on by a variety of reasons including illness, such as diabetes. The purpose of monitoring the blood glucose concentration level is to determine the blood glucose concentration level and then to take corrective action, based upon whether the level is too high or too low, to bring the level back within a normal range. The failure to take corrective action can have serious implications. When blood glucose levels drop too low, a condition known as hypoglycemia, a person can become nervous, shaky, and confused. That person's judgment may become impaired and that person may eventually pass out. A person can also become very ill if their blood glucose level becomes too high, a condition known as hyperglycemia. Both conditions, hypoglycemia and hyperglycemia, are potentially life-threatening emergencies.

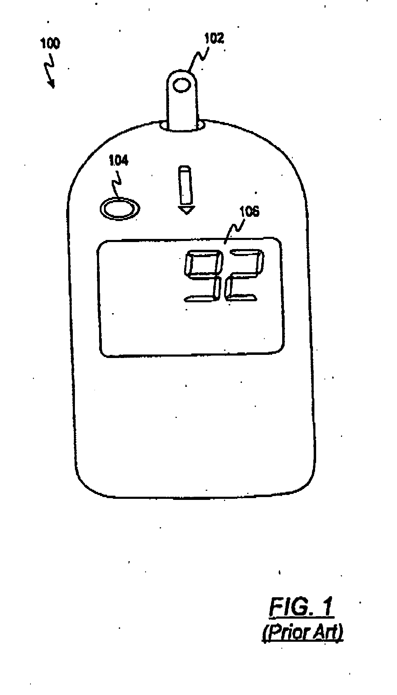

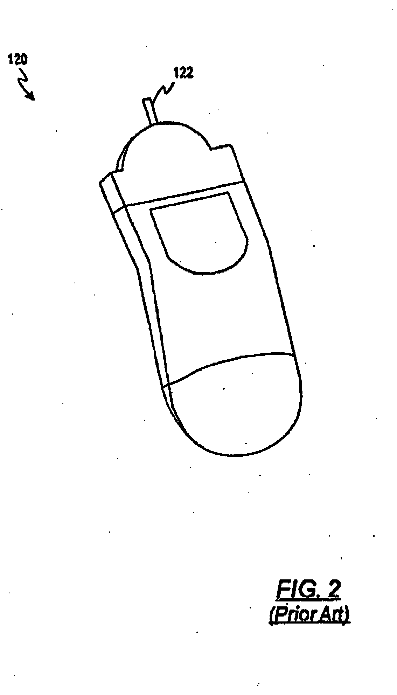

One method of monitoring a person's blood glucose level is with a portable, handheld blood glucose testing device. A prior art blood glucose testing device 100 is illustrated in FIG. 1. The portable nature of these devices 100 enables the users to conveniently test their blood glucose levels wherever the user may be. The glucose testing device contains a test sensor 102 to harvest the blood for analysis. The device 100 contains a switch 104 to activate the device 100 and a display 105 to display the blood glucose analysis results. In order to check the blood glucose level, a drop of blood is obtained from the fingertip using a lancing device. A prior art lancing device 120 is illustrated in FIG. 2. The lancing device 120 contains a needle lance 122 to puncture the skin. Some lancing devices implement a vacuum to facilitate drawing blood. Once the requisite amount of blood is produced on the fingertip, the blood is harvested using the test sensor 102. The test sensor 102, which is inserted into a testing unit 100, is brought into contact with the blood drop. The test sensor 102 draws the blood to the inside of itself. The test sensor, in combination with the testing unit, then determines the concentration of glucose in the blood. Once the results of the test are displayed on the display 106 of the test device 100, the test sensor 102 is discarded. Each new test requires a new test sensor 102.

One problem associated with current test devices is that the test device comprises a two step operation for sample generation and sample harvesting/reading. The two operations are accomplished with two separate instruments (a lance and a test sensor), each having a separate disposable. This requires more parts and more work for the user in disposing the parts.

Another problem associated with current test devices is the difficulty in harvesting small samples when the test sensor is separate from the lance. There is a trend in glucose testing towards minimizing the sample volume. This trend is based on the assumption that there is a corresponding reduction in pain when less sample volume is acquired. As the sample volume is reduced, it becomes more difficult to manually manipulate the test sensor in order to harvest the blood. This is especially true for people who may have seeing impairments or other disabilities, making it difficult to manipulate the test sensor within a small area.

Another problem associated with obtaining small sample sizes is related to the precision needed to obtain the samples. When only small amounts of blood are produced by the lance, it is important that the entire sample or most of the sample be drawn into the test device. When larger volumes of blood are drawn, it is less necessary to obtain all of the blood for the sensor. In small volume test devices, it is important that the sensor be located very near to the puncture wound to maximize the amount of blood that is drawn into the sensor for testing. In current test devices, where the sensor has to be manually moved to the puncture wound, it may be difficult to get close enough to the wound to obtain enough of the sample.

Some current test devices utilize an integrated sensor and lance. The lance is perpendicular to the plane of the test sensor and penetrates through the sensor surface. These sensors, however, still experience the problem that the test sensor must be manually manipulated after the lancing operation is performed.

Another test device has been developed for the collection of interstitial fluid (ISF) that utilizes an integrated lance and reaction area. ISF is collected by piercing just below the skin before any nerve endings or any capillaries. Collecting ISF is sometimes desirable because there is no pain involved since it is above any nerve endings. The lance in this test device is not strong enough to pierce through the dermal layer of the skin in order to obtain samples of other fluids, such as blood. One disadvantage of this and other integrated systems is that the user is forced to dispose of the lance with each test device, an additional expense, as most users reuse their lancets a number of times. A second disadvantage is that any reagent in the device is necessarily exposed to extreme conditions during the required sterilization of the lance. Such exposure may affect the performance of the device.

GB 2 331 935 A discloses a cartridge comprising a cartridge case housing, an extendable lancet and, associated with the case, a compartment for receiving blood which includes an analytical region for analysing a property of the blood. The analytical region may be proximate to the lancing opening and the cartridge may also comprise a reagent which interacts with blood. The cartridge may be of flat or bar-shaped form. In an alternative embodiment, a blood-sampling cartridge comprises a cartridge case and, associated therewith, an absorbent surface for wiping blood off the skin and a compartment for receiving blood. The lancet is mounted in the cartridge case in such a manner that (1) it can move with respect to the cartridge case and extend through the opening when forced by a separate actuator, and (2) when no actuating force acts on the test cartridge, the lancet has a natural resting position entirely inside the cartridge case. In one embodiment the analytical region resides directly neighboring the lancet near the tip, so that the entrance to the capillary volume and the exit port for the lancet are nearly coincident.

### SUMMARY OF THE INVENTION

The present invention is a fluid collection apparatus adapted to test a concentration of an analyte in a fluid and includes a lid and a base. The fluid collection apparatus further includes a spacer disposed between the lid and the base. The spacer forms a capillary channel, which has an opening and is designed to collect the fluid,- An elongated lancing channel is also disposed between the lid and the base having an end adjacent to the opening of the capillary channel. A lance is disposed in the lancing channel at an acute angle relative to the capillary channel and the lance is moveable relative to the base and parallel to the plane of the base between a first position and a second position, in said second position an end of the lance extending beyond the lid and the base for puncturing and the end of the lance being adjacent the opening of the capillary channel.

The capillary channel may also includes a reagent that reacts with the fluid to produce a measurable reaction. The reaction will indicate the concentration of the name in the fluid.

The above summary of the present invention is not intended to represent each embodiment, or every aspect, of the present invention. This is the purpose of the figures and the detailed description which follow.

### BRIEF DESCRIPTION OF THE DRAWINGS

The foregoing and other advantages of the invention will become apparent upon reading the following detailed description and upon reference to the drawings.
FIG. 1 is a top view of a prior art blood glucose testing device.
FIG. 2 is a perspective view of a prior art lance.
FIG. 3 is a top end view of a test device according to one embodiment not being part of the present invention.
FIG. 4a is a front view of a test device having a cover removed according to one embodiment not being part of the present invention.
FIG. 4b is a front view of a test device having a cover removed according to another embodiment not being part of the present invention.
FIG. 5 is a side view of a test device according to one embodiment not being part of the present invention.
FIG. 6 is a top view of the test device of FIG. 5.
FIG. 7 is a top view of the test device according to the embodiment of the present invention.

While the invention is susceptible to various modifications and alternative forms, specific embodiments have been shown by way of example in the drawings and will be described in detail herein. It should be understood, however, that the invention is not intended to be limited to the particular forms disclosed. Rather, the invention is to cover all modifications, equivalents, and alternatives falling within the scope of the invention as defined by the appended claims.

### DESCRIPTION OF ILLUSTRATIVE EMBODIMENTS

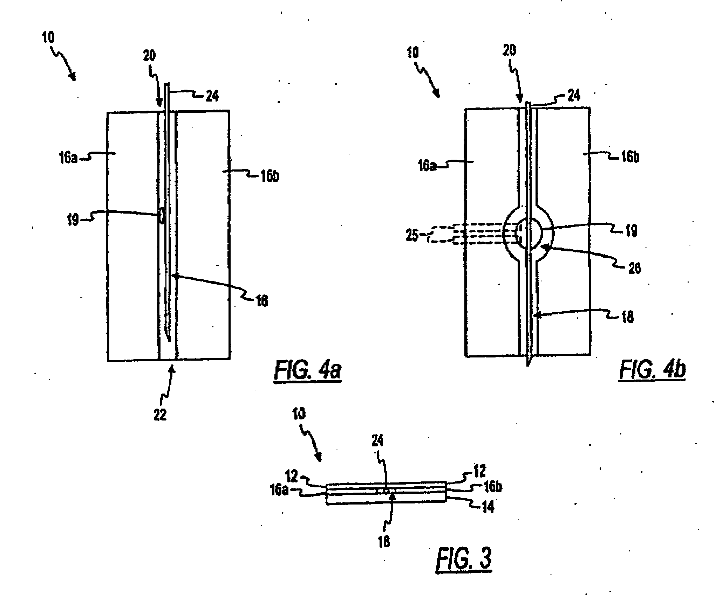

FIG. 3 depicts a fluid collection apparatus 10 according to an embodiment not being part of the present invention. The fluid collection apparatus 10 is designed to collect a fluid, such as blood, so the fluid may be tested for the concentration of a particular analyte, such as glucose. In describing the details of the operation of the fluid collection apparatus 10, the fluid described will be blood pricked from a finger and the analyte will be glucose. It is understood that the embodiment may also be used for other fluids and analytes and that these only serve as examples.

The fluid collection apparatus 10 includes a lid 12, a base 14, and a pair of spacers 16a, 16b disposed between the lid 12 and the base 14. The pair of spacers 16a, 16b form a capillary channel 18. In the illustrated embodiment, the capillary channel 18 is elongated and spans the entire length of the spacers (shown in FIG. 4a). The capillary channel 18 has a first end 20 and a second end 22 (shown in FIG. 4a). The capillary channel 18 includes a reagent 19 that will react with the drawn blood in order to create a measurable reaction. According to one embodiment, the reagent 19 is disbursed throughout the entire capillary channel. A lance 24 is disposed in the capillary channel 18. The lance 24 is moveable through the capillary channel 18 in a direction parallel to the length of the capillary channel 18.

In one embodiment, the fluid collection apparatus 10 can be used in conjunction with a photometric test device to measure the concentration of the analyte directly, for example, by the absorption of light in the infrared region. The test device would measure the amount of infrared light absorbed. Alternatively, a reagent 19 can be used that causes a change in color in the capillary channel. The photometric test device then reads the amount of color change. Photometric testing is described in more detail in commonly-owned U.S. Patent No. 5,611,999 entitled "Diffuse Reflectance Readhead". It is also contemplated that other methods of measuring the concentration of glucose in blood may be utilized.

In another embodiment of the fluid collection apparatus 10, an electrochemical test device is employed as shown in FIG. 4b. The capillary channel 18 includes a pair of electrodes 25. In electrochemical analysis, the change in current across the electrodes 25 caused by the reaction of the glucose and the reagent 19 creates an oxidation current at the electrodes 25 which is directly proportional to the user's blood glucose concentration. The current can be measured by an electrochemical test device coupled to a pair of terminals (not shown) corresponding to the electrodes 25. The electrochemical test device can then communicate to the user the blood glucose concentration. An example of an electrochemical test system is described in detail by commonly-owned U.S. Patent No. 5,723,284 entitled "Control Solution And Method For Testing The Performance Of An Electrochemical Device For Determining The Concentration Of An Analyte In Blood".

Turning now to FIG. 4a, a top view of the fluid collection apparatus 10 with the lid 12 removed is shown. As can be seen in this view, the lance 24 extends through the capillary channel 18 and out of the first end 20. The reagent 19 may be placed anywhere within the capillary channel 18.

In FIG. 4b, an alternative embodiment of the fluid collection apparatus 10 is shown. In this embodiment, the capillary channel 18 includes a detection area 26. The detection area 26 may be a reaction area that includes the reagent 19 and is slightly wider than the rest of the capillary channel 18. The enlarged area makes viewing easier and is used with some optical sensors.

In one embodiment, the capillary channel 18 is from approximately 0.020 to approximately 0.040 inches in length and from approximately 0.006 to approximately 0.012 inches in width. The lance 24 is from approximately 0.005 to approximately 0.011 inches in diameter. The detection area 26 has an area of approximately 0.7 x 10⁻³ to approximately 10 x 10⁻³ inches squared.

The operation of the device 10 illustrated in the embodiments of FIGS. 3-4b will now be described. A user will position the apparatus such that the second end 22 of the capillary channel 18 is pressed against the skin. The lance 24 is in a first position, shown in FIG. 4a, extending out from the first end 20 of the capillary channel 18. The user then pushes the lance 24 downward to a second position shown in FIG. 4b, such that the lance 24 extends past the second opening 22 of the capillary channel 18 and enters the skin. The lance 24 is pushed downward with enough force to create a puncture wound sufficient to draw blood. The lance 24 has a length greater than the capillary channel 18, allowing the lance 24 to extend past both the first and the second ends 20, 22 of the capillary channel 18. Once the lance 24 has punctured the skin, the user pulls lance 24 out of the skin and up the capillary channel 18, at least past the reaction area 26. Blood is drawn into the capillary channel 18 via capillary action. The reagent 19 in the capillary channel 18 reacts with the blood to create a reaction that can be measured as discussed above. In some embodiments, the capillary channel 18 includes stops (not shown) that prevent the lance 24 from being completely pulled out of the capillary channel 18. In these embodiments, it is only necessary to pull the lance past the location of the reagent 19.

The fluid collection apparatus 10 as described provides the advantage of placing the harvesting or collection point of the sensor at the same location as the puncture wound from the lance 24. This eliminates the need to move the fluid collection apparatus 10 around after drawing blood in order to harvest the blood. The device 10 is easier to use, because the users will not have to manually manipulate the sensor after the puncture by trying to place the sensor at the precise location of the puncture.

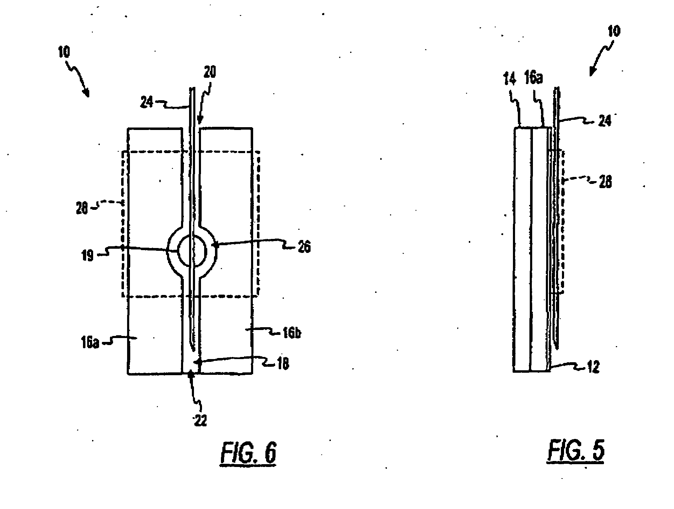

Turning now to FIGS. 5 and 6, another embodiment not being part of the present invention will be shown. Like reference numerals will be used to identify like structures. In this embodiment, the fluid collection apparatus 10 includes the base 14, the pair of spacers 16a, 16b, the capillary channel 18 that is defined by the spacers 16a, 16b, the lid 12, and the lance 24. Alternatively, the base and the spacers or the lid and the spacers can be combined into a single piece that has been molded or formed to this three dimensional shape. In the embodiment shown in FIG. 6, the fluid collection apparatus 10 includes a detection area 26. The detection area 26 may be a specific reaction area including the reagent 19. Alternatively, the reagent 19 is dispersed throughout the entire capillary channel 18. In another embodiment, there is no reagent and an infrared detector may be used to measure the absorption of infrared light.

The collection apparatus 10 also includes a guide 28 for moving the lance. The guide 28 is slidably engaged to the base 14, the spacers 16a, 16b, or the lid 12. The guide 28 is moveable in a direction parallel to the length of the capillary channel 18. The guide 28 is attached to the lance 24. In this embodiment, the lance 24 is not disposed inside the capillary channel 18 but, instead, is adjacent to the capillary channel 18.

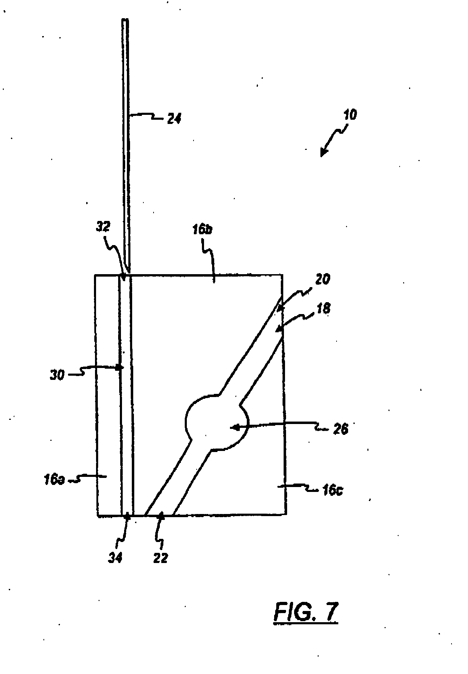

The lance 24 is disposed so that it will draw blood at a location adjacent to the second end 22 of the capillary channel 18. The lance 24 may be located at an angle relative to the capillary channel 18 (such as shown in another alternative embodiment depicted in FIG. 7) or it may be located directly above the capillary channel 18 (shown in FIGS. 5 and 6). Other embodiments are contemplated having the second end 22 of the capillary channel 18 adjacent to the puncture wound, but having different orientations for the lance 24 and the capillary channel 18.

Returning now to the description relating to FIGS. 5 and 6, the guide 28 is used to move the lance 24 between the first and second positions shown in FIGS. 4a and 4b. When in the second position, the lance will pierce the skin for drawing blood, creating a puncture wound. Because the second end 22 of the capillary channel 18 is adjacent to the puncture wound, blood will flow from the wound into the capillary channel 18 via capillary action without any manual moving of the fluid collection apparatus 10. In this embodiment, the lance 24 only needs to be pulled out of the skin, but does not need to be pulled completely out of the capillary channel 18, since the lance's location will not prevent the blood from entering the capillary channel 18. Since the guide 28 is wider than the lance 24, the guide 28 may be easier for some users to grasp and use than the prior embodiment.

Turning now to FIG. 7, the embodiment of the present invention will be described. In this embodiment, the lance 24 is disposed in a lance channel 30, having a first end 32 and a second end 34. The lance channel 30 is formed by first and second spacers 16a, 16b. The capillary channel 18 is formed by the second spacer 16b and a third spacer 16c. The lance 24 is moveable within the lance channel 30 in a direction parallel to the length of the channel 30. The lance channel 30 is disposed such that the second end 34 of the lance channel 30 is adjacent to the second end 22 of the capillary channel 18.

In operation, the fluid collection apparatus 10 is placed against the skin as in the other embodiments. The lance 24 is then pushed downward through the lance channel 30 and into the skin. After the skin is punctured, the lance 24 is withdrawn from the skin, but remains within the lance channel 30. The blood is then drawn into the capillary channel 18, via capillary action. By keeping the lance 24 in the lance channel 30, the lance channel 30 is not able to draw any blood into it, and all of the blood is instead drawn into the adjacent capillary channel 18. Alternatively, at least one face of the lance channel 30 can be of a hydrophobic material that inhibits entry of the blood into the lance channel 30.

In the illustrated embodiment, the capillary channel 18 includes the detection area 26. In this embodiment, the reagent 19 is kept in the detection area 26, creating the measurable reaction in the detection area 26. In some embodiments, there will not be a specific, enlarged detection area 26 and the reagent 19 will be dispersed elsewhere in the capillary channel 18.

While the present invention has been described with reference to the embodiment of Fig. 7 , those skilled in the art will recognize that many changes may be made thereto without departing from the scope of the present invention, which is set forth in the following claims.

## Claims

1. A fluid collection apparatus (10) to test a concentration of an analyte in a fluid, comprising:
a lid (12);
a base (14) having a plane:
a spacer (16a, 16b) disposed between said lid (12) and said base (14), said spacer (16a, 16b) including a capillary channel (18) having an opening (22) for receiving the fluid;
an elongated lancing channel (30) disposed between said lid (12) and said base (14) ;
a lance (24) disposed in said lancing channel (30) and
said lance (24) being moveable relative to said base (14) and parallel to said plane of said base (14) between a first position and a second position,
in said second position an end of said lance (24) extending beyond said lid (12) and said base (14) for puncturing;
**characterized in that** said lance (24) is disposed at an acute angle relative to said capillary channel (18), **in that** an end of said elongated lancing channel (30) is adjacent said opening (22) of the capillary channel (18) and **in that** in said second position said end of the lance (24) is adjacent said opening (22) of the capillary channel (18)..̅

2. The fluid collection apparatus (10) of claim 1, wherein said lancing channel (30) includes at least one face, said at least one face of said lancing channel (30) is of a hydrophobic material.

3. The fluid collection apparatus (10) of claim 1 or 2, wherein said capillary channel (18) has a length of approximately 0.508 (0.020) to approximately 1.016 mm (0.040 inches).

4. The fluid collection apparatus (10) of one of the claims 1 to 3, wherein said capillary channel (18) includes a detection area (26) for containing a reagent (19) adapted to produce a reaction indicative of the concentration of the analyte in the fluid.

5. The fluid collection apparatus (10) of claim 4, wherein said detection area (26) has an area of approximately 0.45 (0.7 x 10⁻³) to 6.45 mm² (10 x 10⁻³ inches squared).

6. The fluid collection apparatus (10) of one of the claims 1 to 5, wherein said lancing channel (30) has a width of approximately 0.15 (0.006) to approximately 0.3 mm (0.012 inches).

7. The fluid collection apparatus (10) of one of the claims 1 to 6, wherein said lance (24) has a diameter of approximately 0.13 mm (0.005) to approximately 0.28 mm (0.011 inches).

8. The fluid collection apparatus (10) of one of the claims 1 to 7, further comprising a reagent (19) disposed in said capillary channel (18) and adapted to produce a reaction indicative of the concentration of the analyte in the fluid.

9. The fluid collection apparatus (10) of claim 8, wherein the reagent (19) is adapted to produce a colorimetric reaction.

10. The fluid collection apparatus (10) of claim 9, in combination with a colorimetric test device.

11. The fluid collection apparatus (10) of claim 10, wherein the reagent (19) is adapted to produce an electrochemical reaction.

12. The fluid collection apparatus (10) of claim 11, in combination with an electrochemical test device.

13. The fluid collection apparatus (10) of one of the claims 1 to 12, wherein the analyte is glucose.

14. The fluid collection apparatus (10) of claim 13, in combination with a test device adapted to measure the concentration of glucose in blood.

15. The fluid collection apparatus (10) of one of the claims 1 to 14, in combination with a test device adapted to measure the absorption of infrared light by the fluid.

## Patentansprüche

1. Fluidsammelvorrichtung (10) zum Testen der Konzentration eines Analyts in einem Fluid, umfassend:
einen Deckel (12);
eine Basis (14) mit einer Ebene;
einen Abstandshalter (16a, 16b), der zwischen dem Deckel (12) und der Basis (14) angeordnet ist, wobei der Abstandshalter (16a, 16b) einen Kapillarkanal (18) mit einer Öffnung (22) zur Aufnahme des Fluids umfasst;
einen länglichen Lanzettenkanal (30), der zwischen dem Deckel (12) und der Basis (14) angeordnet ist;
eine Lanzette (24), die in dem Lanzettenkanal (30) angeordnet ist, wobei die Lanzette (24) in Bezug auf die Basis (14) und parallel zu der Fläche der Basis (14) zwischen einer ersten Position und einer zweiten Position bewegbar ist, wobei sich in der zweiten Position ein Ende der Lanzette (24) zum Punktieren über den Deckel (12) und die Basis (14) hinaus erstreckt;
**dadurch gekennzeichnet, dass** die Lanzette (24) in Bezug auf den Kapillarkanal (18) in einem spitzen Winkel angeordnet ist, dass ein Ende des länglichen Lanzettenkanals (30) benachbart zur Öffnung (22) des Kapillarkanals (18) liegt und dass in der zweiten Position das Ende der Lanzette (24) benachbart zur Öffnung (22) des Kapillarkanals (18) liegt.

2. Fluidsammelvorrichtung (10) nach Anspruch 1, worin der Lanzettenkanal (30) zumindest eine Fläche umfasst, wobei die zumindest eine Fläche des Lanzettenkanals (30) aus einem hydrophoben Material besteht.

3. Fluidsammelvorrichtung (10) nach Anspruch 1 oder 2, worin der Kapillarkanal (18) eine Länge von etwa 0,508 mm (0,020 Zoll) bis etwa 1,016 mm (0,040 Zoll) aufweist.

4. Fluidsammelvorrichtung (10) nach einem der Ansprüche 1 bis 3, worin der Kapillarkanal (18) einen Detektionsbereich (26) zur Aufnahme eines Reagens (19) umfasst, das geeignet ist, eine Reaktion hervorzurufen, die ein Hinweis auf die Konzentration des Analyts im Fluid ist.

5. Fluidsammelvorrichtung (10) nach Anspruch 4, worin der Detektionsbereich (26) eine Fläche von etwa 0,45 mm² (0,7 x 10⁻³ Quadratzoll) bis 6,45 mm² (10 x 10⁻³ Quadratzoll) aufweist.

6. Fluidsammelvorrichtung (10) nach einem der Ansprüche 1 bis 5, worin der Lanzettenkanal (30) eine Breite von etwa 0,15 mm (0,006 Zoll) bis etwa 0,3 mm (0,012 Zoll) aufweist.

7. Fluidsammelvorrichtung (10) nach einem der Ansprüche 1 bis 6, worin die Lanzette (24) einen Durchmesser von etwa 0,13 mm (0,005 Zoll) bis etwa 0,28 mm (0,011 Zoll) aufweist.

8. Fluidsammelvorrichtung (10) nach einem der Ansprüche 1 bis 7, die weiters ein Reagens (19) umfasst, das im Kapillarkanal (18) angeordnet ist und geeignet ist, eine Reaktion hervorzurufen, die ein Hinweis auf die Konzentration des Analyts im Fluid ist.

9. Fluidsammelvorrichtung (10) nach Anspruch 8, worin das Reagens (19) geeignet ist, eine kolorimetrische Reaktion hervorzurufen.

10. Fluidsammelvorrichtung (10) nach Anspruch 9 in Kombination mit einer kolorimetrischen Testvorrichtung.

11. Fluidsammelvorrichtung (10) nach Anspruch 10, worin das Reagens (19) geeignet ist, eine elektrochemische Reaktion hervorzurufen.

12. Fluidsammelvorrichtung (10) nach Anspruch 11 in Kombination mit einer elektrochemischen Testvorrichtung.

13. Fluidsammelvorrichtung (10) nach einem der Ansprüche 1 bis 12, worin der Analyt Glucose ist.

14. Fluidsammelvorrichtung (10) nach Anspruch 13 in Kombination mit einer Testvorrichtung, die zur Messung der Konzentration von Glucose in Blut geeignet ist.

15. Fluidsammelvorrichtung (10) nach einem der Ansprüche 1 bis 14 in Kombination mit einer Testvorrichtung, die zur Messung der Absorption von Infrarotlicht durch das Fluid geeignet ist.

## Revendications

1. Appareil de prélèvement de fluide (10) destiné à tester la concentration d'un analyte dans un fluide, comprenant :
un couvercle (12) ;
un socle (14) ayant un plan ;
un élément d'espacement (16a, 16b) disposé entre ledit couvercle (12) et ledit socle (14), ledit élément d'espacement (16a, 16b) comportant un canal capillaire (18) présentant une ouverture (22) destinée à recevoir le fluide ;
un canal de piquage oblong (30) disposé entre ledit couvercle (12) et ledit socle (14) ; une lancette (24) disposée dans ledit canal de piquage (30) et
ladite lancette (24) étant mobile par rapport audit socle (14) et parallèle audit plan dudit socle (14) entre une première position et une deuxième position,
à ladite deuxième position, une extrémité de ladite lancette (24) s'étendant au-delà dudit couvercle (12) et dudit socle (14) pour piquer ;
**caractérisé en ce que** ladite lancette (24) est disposée en formant un angle aigu par rapport audit canal capillaire (18), **en ce qu'**une extrémité dudit canal de piquage oblong (30) est adjacente à ladite ouverture (22) dudit canal capillaire (18) et **en ce que**, à ladite deuxième position, ladite extrémité de la lancette (24) est adjacente à ladite ouverture (22) du canal capillaire (18).

2. Appareil de prélèvement de fluide (10) selon la revendication 1, dans lequel ledit canal de piquage (30) comprend au moins une face, ladite au moins une face dudit canal de piquage (30) étant constituée d'un matériau hydrophobe.

3. Appareil de prélèvement de fluide (10) selon la revendication 1 ou 2, dans lequel ledit canal capillaire (18) a une longueur d'environ 0,508 mm (0,020 pouce) à environ 1,016 mm (0,040 pouce).

4. Appareil de prélèvement de fluide (10) selon l'une des revendications 1 à 3, dans lequel ledit canal capillaire (18) comprend une zone de détection (26) destinée à contenir un réactif (19) apte à produire une réaction représentative de la concentration de l'analyte dans le fluide.

5. Appareil de prélèvement de fluide (10) selon la revendication 4, dans lequel ladite zone de détection (26) a une superficie d'environ 0,45 mm² (0,7 x 10⁻³ pouce carré) à 6,45 mm² (10 x 10⁻³ pouce carré).

6. Appareil de prélèvement de fluide (10) selon l'une des revendications 1 à 5, dans lequel ledit canal de piquage (30) a une largeur d'environ 0,15 mm (0,006 pouce) à environ 0,3 mm (0,012 pouce).

7. Appareil de prélèvement de fluide (10) selon l'une des revendications 1 à 6, dans lequel ladite lancette (24) a un diamètre d'environ 0,13 mm (0,005 pouce) à environ 0,28 mm (0,011 pouce).

8. Appareil de prélèvement de fluide (10) selon l'une des revendications 1 à 7, comprenant en outre un réactif (19) disposé dans ledit canal capillaire (18) et apte à produire une réaction représentative de la concentration de l'analyte dans le fluide.

9. Appareil de prélèvement de fluide (10) selon la revendication 8, dans lequel le réactif (19) est apte à produire une réaction colorimétrique.

10. Appareil de prélèvement de fluide (10) selon la revendication 9, combiné à un dispositif de test colorimétrique.

11. Appareil de prélèvement de fluide (10) selon la revendication 10, dans lequel le réactif (19) est apte à produire une réaction électrochimique.

12. Appareil de prélèvement de fluide (10) selon la revendication 11, combiné à un dispositif de test électrochimique.

13. Appareil de prélèvement de fluide (10) selon l'une des revendications 1 à 12, dans lequel l'analyte est le glucose.

14. Appareil de prélèvement de fluide (10) selon la revendication 13, combiné à un dispositif de test apte à mesurer la concentration du glucose dans le sang.

15. Appareil de prélèvement de fluide (10) selon l'une des revendications 1 à 14, combiné à un dispositif de test apte à mesurer l'absorption de lumière infrarouge par le fluide.
